(19) Europäisches Patentamt
European Patent Office
Office européen des brevets

(11) Publication number: 0 491 218 A1

# (12) EUROPEAN PATENT APPLICATION

(21) Application number: 91120709.0

(22) Date of filing: 03.12.91

(51) Int. Cl.[5]: C07D 403/04, C07D 207/32, C07D 231/12, C07D 233/54, C07D 249/04, C07D 257/04

(30) Priority: 17.12.90 US 628551

(43) Date of publication of application: 24.06.92 Bulletin 92/26

(84) Designated Contracting States: AT BE CH DE DK ES FR GB GR IT LI LU MC NL SE

(71) Applicant: F. HOFFMANN-LA ROCHE AG
Postfach 3255
CH-4002 Basel(CH)

(72) Inventor: Hsu, Ming-Chu
445 East 86th Street, Apt.15 G.
New York, N.Y. 10028(US)
Inventor: Huryn, Donna Mary
164 Hillcroft Way
Newtown, Pennsylvania 18940(US)
Inventor: Tam, Steve Yik-Kai
13 Evergreen Road
West Caldwell, N.J. 07006(US)

(74) Representative: Mahé, Jean et al
Postfach 3255 Grenzacherstrasse 124
CH-4002 Basel(CH)

## (54) Benzodiazepinones.

(57) Novel benzodiazepinones of the formula

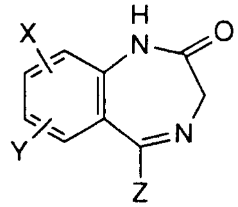

I

and compositions containing same for alleviation of viral infections. including HIV infections.

EP 0 491 218 A1

The present invention relates to benzodiazepinones of formula

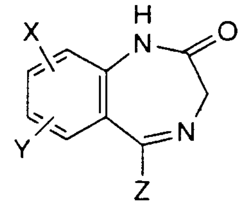

I

wherein X and Y are H, $NO_2$, $NR^1R^2$, halogen, $CF_3$, OH, cyano or lower alkyl or alkoxy;
$R^1$ and $R^2$ are H, lower alkyl, aryl or aralkyl; and
Z is a ring of one of the formulae:

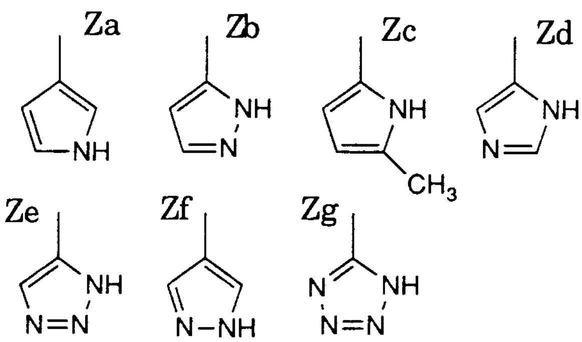

Za Zb Zc Zd Ze Zf Zg

wherein one or more C-atoms can be substituted by H, F, Cl or lower alkyl;
and pharmaceutically acceptable salts, esters and amides thereof.

Objects of the present invention are the above compounds per se and for use as a therapeutically active agent, especially for the treatment or prophylaxis of viral infections, particularly of retroviral infections, such as HIV 1 and/or HIV 2 infections, or for protecting cells against such infections;

further a process for the manufacture of these compounds and medicaments containing one of such compounds and, optionally, a second antiviral agent, especially a reverse transcriptase inhibitor, such as ddC, AZT or ddI, TIBO derivatives, tricyclic diazepinones, a HIV-protease inhibitor, $\alpha$-, $\beta$- and/or $\gamma$-interferon, interleukin-2 and/or GM-CSF, and

the use of these compounds for the manufacture of medicaments especially for the treatment or prophylaxis of viral infections, particularly of retroviral infections, such as HIV 1 and/or HIV 2 infections, or for protecting cells against such infections.

As used therein, the term "lower" refers to straight or branched hydrocarbon chains with up to 7 C atoms, such as methyl, ethyl, propyl and isopropyl. Examples of "aryl" groups, taken alone or in combination, e.g. in "aralkyl" are phenyl, tolyl and xylyl. "Acyl" groups are derived from aliphatic or aromatic acids, e.g. acetic, sulfonacetic or benzoic acid.

All the tautomeric and stereoisomeric forms of the compounds of formula I are included in the scope of this invention.

Pharmaceutically acceptable salts may be those with organic acids, e.g. lactic, acetic, malic or p-toluenesulfonic acid; or salts with mineral acids, such as hydrochloric or sulfuric acid.

Preferred compounds of formula I are those wherein X is H, Y is Cl, particularly Cl in the 7-position, and Z is $Z^c$, $Z^d$ or, particularly, $Z^a$, $Z^b$ or $Z^e$.

The compounds of the invention can be prepared in a manner known per se, e.g. as described in US 3405122, 3398159, 3407211 and 3400128; in J. Org. Chem. 41, 1976, 2720; 35, 1970, 2455 and 46, 1981, 839; in Acta Chem. Scan. B 31, 1977, 701; in J. Heterocyclic Chem. 12, 1975, 49 and 25, 1988, 1293; in Synthesis 1988, 767; in Syn. Commun. 15, 1985, 1271 and J.A.C.S. 100, 1978, 4842.

Thus the compounds of the invention can be prepared by cyclizing a compound of formula

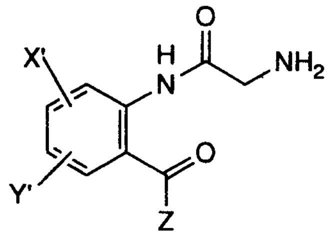

V

wherein X' and Y' are H, $NO_2$, halogen, $CF_3$ or lower alkyl; and Z is as above, by acid-catalysis.

This cyclization can be performed by heating the compound V with an acid, such as pivalic acid, in a solvent, such as toluene and THF, or in n-butanol, at a temperature up to reflux temperature.

The amines V can be prepared via the corresponding bromides IV

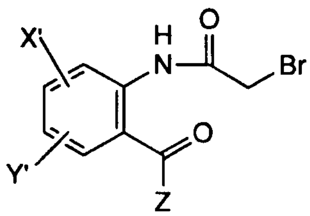

IV

starting from ketones of formula III

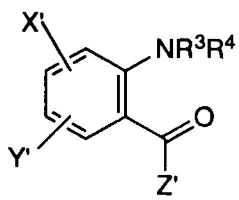

III

wherein X' and Y' are as above,

Z' is selected from the group consisting of

Z'a

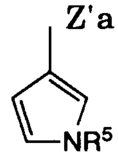

Z'b

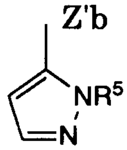

Z'c

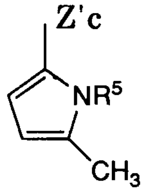

Z'd

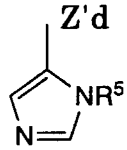

Z'e

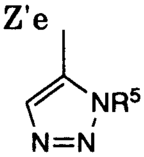

Z'f

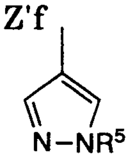

Z'g

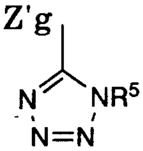

where $R^3$, $R^4$ and $R^5$ are H or a N-protecting group.

Suitable N-protecting groups include acyl, trialkylsilyl, alkyldiarylsilyl, ethoxymethyl, (dialkylamino)-methyl, t-butoxycarbonyl and phenoxycarbonyl. A preferred method for performing the reaction III→IV→V involves bromoacetylation of a ketone III to the bromide IV, followed by ammonolysis to the amine V.

The ketones III can be prepared by reacting a metalloheterocycle of formula VI

$R^6$-Z''    VI

where Z'' is the same as Z' above except that one or more hydrogens on the C-atoms in Z' may be replaced by an unreactive blocking group; and $R^6$ is a metal or a metallic halide group, such as MgBr, MgCl, Li, Na or Sn,
with an aromatic compound of formula VII

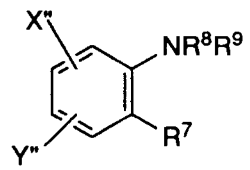

VII

where $R^7$ is formyl or a functional derivative of a carboxylic acid, such as cyano, ester, amide or acyl chloride, X'' and Y'' are defined as X' and Y' above except that X'' and Y'' cannot be $NO_2$ and $R^8$ and $R^9$ are H, O, acyl, trialkylsilyl, alkyldiarylsilyl or t-butoxycarbonyl.

In formula VI above, preferable unreactive blocking groups include halogen, S-lower alkyl, S-aryl, trialkylsilyl and alkyldiarysilyl.

When $R^7$ is formyl, an alcohol of formula VIII is generated

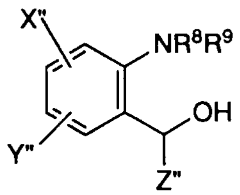

VIII

where X'',Y'', Z'', $R^8$, and $R^9$ are as defined above.

When $R^7$ in formula VII is other than formyl, a ketone of formula IX is obtained

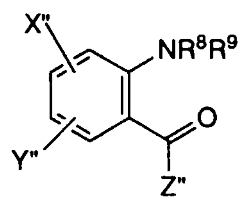

IX

where X'', Y'', Z'', $R^8$ and $R^9$ are as defined above.

The conversion of VIII to IX can be accomplished by oxidation, e.g. catalytic oxydation or reaction with active maganese dioxide.

Compound IX is then converted to the desired compound of formula III using conventional methods, e.g. as described in the Examples.

Similarly, a compound of formula VI is reacted with a compound of formula X

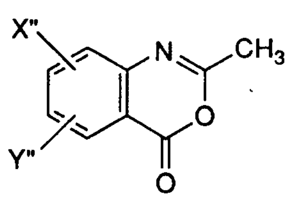

X

where X'' and Y'' are as above, to generate the desired ketone IX wherein $R^8$ is H and $R^9$ is acetyl.

The ketones III, wherein Z is a group Zb or Ze, can also be prepared by a dipolar cycloaddition reaction

of a compound of formula XI

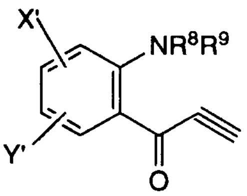

XI

where $R^8$, $R^9$, X' and Y' are as above,
with an azide, diazomethane or a derivative thereof.

Compounds XI can be prepared via addition of a metallic-ethynyl reagent to a compound of formula VII or X.

A ketone III wherein Z' is the group Za, can also be synthesized by acid-induced rearrangement of an isomeric 2-acyl-heterocycle compound of formula XII below, as follows:

XII

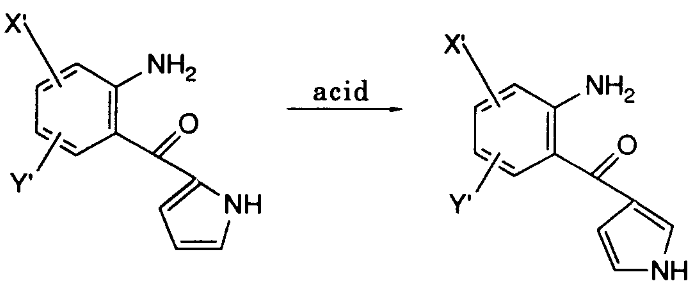


XIII

wherein X' and Y' are as defined above.

Useful acids include trifluoromethanesulfonic acid and polyphosphoric acid.

The introduction of substituents OH, O-alkyl, $NH_2$ and cyano onto the benzene ring as X and Y can be accomplished by common reactions known in the art. Similarly, the interconversion of nitro to cyano or to amines and of amines to $NR^1R^2$, where $R^1$ and $R^2$ are as above, as well as the conversion of alkoxy to OH are well-known in the art.

The compounds of formulae III, IV' (i.e. IV and V) and VIII' (i.e. VIII and IX) are novel and as such object of the present invention.

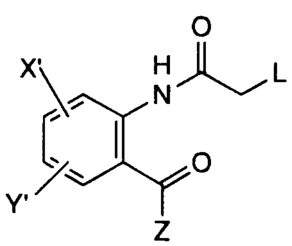

IV' and

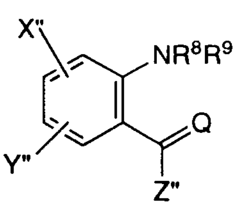

VIII'

The compounds I and their salts have useful antiviral, especially anti-retroviral activity, particularly against HIV, the virus implicated in the development of AIDS and related diseases such as ARC (AIDS related complex). These compounds also inhibit HIV replication by inhibiting such important HIV viral functions as TAT (transactivating transcriptional) activity.

The compounds of formula I were tested for anti-HIV-TAT activity in an assay comprising the following steps:

(a) putting both the expression of the Secreted Alkaline Phosphatase (SeAP) gene and the viral transactivator TAT gene under the control of the HIV promoter LTR responsive to the action of the HIV transactivator TAT;

(b) transfecting cultured mammalian cells with plasmids which contain the gene constructs of (a) above

and cause cellular production of the transactivating factor TAT and SeAP;

(c) adding the agent to be tested, here the compounds of formula I; and determining the amount of SeAP produced, by measuring SeAP enzymatic activity, whereby inhibition of SeAP production correlates with the anti-TAT inhibition activity.

In this assay, the inhibition of SeAP positively correlates with anti-TAT activity. The greater the ability of an agent to inhibit SeAP, the greater is its anti-TAT activity.

Specifically, with respect to the results reported below, the anti-HIV-TAT assay was run as follows:

At 24 hours post transfection 1, 10, 25 and 50 μM of a test compound of formula I was added to the culture media of COS cells transfected with two plasmids, one containing the reporter gene which codes for SeAP under control of HIV-LTR, and the other containing the HIV-TAT gene also under control of HIV-LTR. The alkaline phosphatase activity of the media was assayed 48 hours after addition of test compound with a colorimetric assay using p-nitrophenylphosphate as the substrate. The anti-TAT activity is measured by the percent inhibition of SeAP gene expression under the control of HIV-LTR versus the percent inhibition of SeAP gene under RSV-LTR, which does not respond to TAT.

The results in the Table below show that the compounds of formula I are specific inhibitors of HIV-TAT-regulated gene expression without non-specific cytotoxic effects.

The specificity of the compounds of formula I as TAT inhibitors was demonstrated with a parallel assay in which the SeAP gene expression is put under control of the Rous sarcoma virus (RSV)-LTR which does not respond to TAT. This assay thus eliminates the possibility that the compounds of formula I are either general cytotoxic agents or inhibit the activity of SeAP.

The anti-HIV-TAT activities of the test compounds were determined by measuring the amount of alkaline phosphatase in the supernatant media of cultures of cells in which SeAP gene expression was under the control of the HIV LTR promoter. The specific inhibitory activities of the test compounds were calculated according to the formula:

$$100 [(1-A/B) - (1-C/D)]$$

where A and B are the alkaline phosphatase activites produced by HIV-LTR/SeAP in the presence and absence, respectively, of test compound, and C and D are the alkaline phosphatase activities produced by RSV-LTR/SeAP in the presence and absence, respectively, of test compound. The concentrations tested ranged from 1-50 μM. The results provided are the average of at least three tests. The test compound was added 24 hours after cells were transfected with the plasmids when SeAP specific mRNA and protein were already present and the protein was very stable. Therefore, 100% inhibition would not be observed with this assay procedure.

Table

| Product of Example No. | Anti-HIV-TAT activity |
|---|---|
| 1 | Medium/High |
| 2 | High |
| 3 | High |
| 4 | Medium/High |
| 5 | Medium/High |

With respect to human patients infected with HIV, and patients with symptomatic or asymptomatic HIV infections, an antivirally-effective amount of a compound of formula I or a salt thereof is in the range of from about 0.5 to 40 mg/kg, preferably from about 1 to 15 mg/kg, more preferably from about 3 to 5 mg/kg body weight per day. In unit dosage form, for a 70 kg patient, this would be an amount of from about 35 to 2800, preferably from about 210 to 350 mg per day. This dosage may be administered parenterally or orally in one or more doses at various intervals daily, preferably orally once daily.

The compounds may also be administered with other antiviral and/or biological response modifiers. For example, the compounds of formula I may be administered with known HIV-RT inhibitors such as ddC, AZT, ddI or non-nucleoside RT inhibitors such as TIBO derivatives or tricyclic diazepinones, or other inhibitors which act against other HIV proteins such as protease, integrase and RNAase, as well as with biological modifiers such as α-, β- or γ-interferon or a combination thereof, interleukin-2 and GM-CSF. The dosages of ddC and AZT used in AIDS or ARC human patients have been published. When given in combined therapy,

the other anti-HIV compounds may be given at the same time as a compound of formula I or the dosing may be staggered as desired. The two (or more) drugs may also be combined in a composition. Doses of each drug may be less when used in combination than when they are used as a single agent.

It is possible for the compounds of the invention to be administered alone in solution. However, it is preferred that the active ingredients be administered in a pharmaceutical formulation or composition. These formulations comprise at least one active ingredient together with one or more pharmaceutically acceptable carrier and excipient and may optionally include other therapeutic agents, for example a protease inhibitor. These carriers include those suitable for oral, rectal, nasal, topical, buccal, sublingual, vaginal or parenteral (including subcutaneous, intramuscular, intravenous and intradermal) administration.

Examples of compositions of the invention are solutions of the active ingredient(s), e.g. in water or saline; capsules, e.g. soft gelatine capsules; sachets or tablets, each containing a pre-determined amount of the active ingredient, e.g. as granules; solutions or suspensions in an aqueous liquid or in an oil-in-water emulsion or a water-in-oil liquid emulsion. Tablets may include one or more of lactose, microcrystalline cellulose, colloidal silicon dioxide, croscarmellose sodium, magnesium stearate, stearic acid and other excipients, colorants and pharmacologically compatible carriers. Formulations suitable for topical administration include lozenges comprising the active ingredient in a flavor, usually sucrose and acacia or tragacanth; pastilles comprising the active ingredient in an inert basis such as gelatin and glycerin, or sucrose and acacia; and mouthwashes comprising the active ingredient in a suitable liquid carrier. Formulations for rectal administration may be presented as a suppository with a suitable base comprising cocoa butter or a salicylate. Formulations suitable for vaginal administration may be presented as pessaries, tampons, creams, gells, pastes, foams or spray formulas. Formulations suitable for parenteral administration include aqueous and non-aqueous, isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example ampules and vials, and may be stored in a lyophilized condition requiring only the addition of the sterile liquid carrier, for example water for injections, immediately prior to use. Extemporaneous injection solutions and suspensions may be prepared from sterile powder, granules and tablets of the kind previously described.

Example 1

a) A solution of 1-(ethoxymethyl)-1H-pyrazole (5.0g) (prepared as in J.A.C.S. 100, 1978, 3918) and THF (200 ml) is treated dropwise under argon, under stirring at -78 °C, with nBuLi (0.039 mole). The resulting mixture is stirred at -78 °C for three hours, then allowed to warm to 0 °C. After re-cooling to -78 °C, a solution of 5-chloro-2-nitro-benzaldehyde (6.0g) in THF (50 ml) is added to the mixture. The mixture is allowed to warm to room temperature overnight, then quenched with saturated $NH_4Cl$ solution (150 ml). The organic layer is separated, and the aqueous layer extracted with EtOAc. The organic layers are dried, then filtered and evaporated. Chromatography using a gradient elution system (20% EtOAc/80% Hexane-50% EtOAc/50% Hexane) yields 4.7 g of alpha-(5-chloro-2-nitrophenyl)-[1- (ethoxymethyl)-1H-pyrazole-5-yl]-methanol, MS 312 ($M^+H$).

b) A mixture of the product of a) (12.2 g), $CHCl_3$ (300 ml) and $MnO_2$ (13.5 g) is heated to 70 °C for three days. After cooling to room temperature, the mixture is filtered and the residue washed with $CHCl_3$. Evaporation of the filtrate yields 11.7 g of (5-chloro-2-nitrophenyl)[1-(ethoxymethyl)-1H-pyrazol-5-yl]-methanone, MS 310 ($M^+H$).

c) A mixture of the product of b) (10.0 g), ethanol (300 ml) and 4N HCl (300 ml) is heated to 60 °C for 3.5 hours. The solution is cooled in an ice-bath, and treated with 4N NaOH dropwise, until slightly basic. The mixture is diluted with $CH_2Cl_2$, the organic layer separated, and the aqueous layer extracted with $CH_2Cl_2$. The organic layers are dried, filtered and evaporated to yield 7.82 g of (5-chloro-2-nitrophenyl)-(1H-pyrazol-5-yl)methanone, MS Calcd: 252.0176, MS Found: 252.0195.

d) A mixture of 2.0 g of the product of c), ethanol (100 ml) and Pd on C (25 mg) is hydrogenated at 35 psi for 12 hours. After filtration of the mixture, the filtrate is evaporated, the residue dissolved in a 20% EtOAc/80% Hexane (50 ml) solution and filtered. After evaporation one obtains 1.15 g of (2-amino-5-chlorophenyl)-(1H-pyrazol-5-yl)-methanone, MS 221 ($M^+$).

e) To a solution of the product of d) (4.12 g) in $CH_2Cl_2$ (460 ml) and THF (50 ml) are added 250 g of ice and 200 ml water. The stirred mixture is treated dropwise with bromoacetyl bromide (45.5 mmole), then stirred for 30 minutes. The two phases are separated, and the organic layer is washed with water and saturated sodium bicarbonate solution, then dried, filtered and evaporated. The product is dissolved in THF (100 ml), then added to liquid $NH_3$ (150 ml) cooled to -78 °C. After stirring overnight while the

ammonia was allowed to evaporate, the residue is partitioned between a 10% MeOH/90% EtOAc solution and water. The aqueous layer is extracted with 10% MeOH/90% EtOAc solution. The organic layers are washed with water and brine, then dried, filtered and evaporated to dryness. This residue is dissolved in toluene and THF. Pivalic acid (500 mg) is added, and the mixture heated to reflux temperature overnight. Evaporation of the solvent affords a solid residue which is slurried with a 5% MeOH/96% $CH_2Cl_2$ solution. The remaining solids are collected to afford 0.64 g of the desired product. An additional 0.9 g of the product are isolated via chromatography of the wash solution using a 4% MeOH/96% $CH_2Cl_2$ solution as eluant. After recrystallization from methanol over charcoal one obtains 7-chloro-5-(1H-pyrazol-5-yl)-1,3-dihydro-2H-1,4-benzodiazepin-2-one, mp 274-276 ° C; MS 260 ($M^+$).

Example 2

a) Acetylene is bubbled into 75 ml of THF. EtMgBr (2.0M) in THF is added to the THF/acetylene mixture over a period of 1 hour such that the temperature of the reaction mixture never exceeds 40 ° C. The mixture is then stirred at room temperature for 30 minutes after which the flow of acetylene is stopped, and the mixture is cooled to 0 ° C. A solution of 5-chloro-2-nitrobenzaldehyde (13.2 g) in 70 ml THF is added dropwise to the mixture. The reaction is allowed to warm to room temperature overnight, then quenched with saturated $NH_4Cl$ solution, and extracted with EtOAc. After drying, filtration and evaporation, the residue is purified via flash chromatography (10% EtOAc/Hexane), to provide 12.26 g of 5-chloro-alpha-ethynyl-2-nitrobenzenemethanol, IR (KBr) 3290, 2120, 1525, 1338 $cm^{-1}$.

b) A solution of the product of a) (1.54 g) in 60 ml glacial acetic acid is heated to 40 ° C, and then treated with 1.44 g $CrO_3$. After stirring, the solution is cooled to room temperature, then extracted with $CH_2Cl_2$. The organic fraction is further extracted with $H_2O$ and saturated $NaHCO_3$, then dried, filtered and evaporated. Chromatography using a gradient elution system (9% EtOAc/Hexane, 17% EtOAc/Hexane) provides 0.94 g of 1-(5-chloro-2-nitrophenyl)-2-propyn-1-one, MS calc. 208.9880; found 208.9872.

c) The product of b) (0.03 g) is mixed with azido-trimethylsilane (0.02 ml) and $CH_3CN$ (5 ml), and the mixture is heated to 100 ° C under argon for three hours. The solution is then evaporated, and subsequently purified via flash column chromatography using a gradient elution system (5% EtOAc/Hexane, 20% EtOAc/Hexane, 50% EtOAc/Hexane) to provide 0.03 g of (5-chloro-2-nitrophenyl)-(1H-1,2,3-triazol-5-yl)methanone, MS 252 ($M^+$).

d) A mixture of the product of c) (2.53 g), EtOH (100 ml) and 10% Pd on C (50 mg) is hydrogenated under 40 psi hydrogen for 16 hours. The solution is then filtered, evaporated and slurried with $CH_2Cl_2$. The resultant solid is collected, yielding 1.64 g of (2-amino-5-chlorophenyl)-(1H-1,2,3-triazol-5-yl)-methanone. The mother liquors are purified by column chromatography using a gradient elution system (9% EtOAc/Hexane, 20% EtOAc/Hexane, 33% EtOAc/Hexane) to provide another 0.48 g of product, mp 148-150 ° C.

e) To a solution of 1.0 g of the product of d) in 150 ml of $CH_2Cl_2$ and 60 ml of THF are added 50 ml of ice water and 2.0 g of sodium bicarbonate. To the stirred mixture are added 0.98 ml of bromoacetyl bromide. The mixture is stirred for 0.5 hours. The layers are separated, and the aqueous portion is extracted with $CH_2Cl_2$. The organic extracts are washed with water, followed by brine, then dried and evaporated. The residue is filtered using ethyl acetate-hexane as eluant to provide 1.2 g of 2-bromo-4'-chloro-2'-[(1H-1,2,3-triazol-5-yl)carbonyl]acetanilide, mp 173-175 ° C.

f) To 100 ml of liquid ammonia in an ice bath is added a solution of 1.2 g of the product of e) in 25 ml of THF. The mixture is stirred overnight and the ammonia allowed to evaporate. The remaining THF is evaporated and the residue is stirred with 10% methanol-ethyl acetate and water. The aqueous portion is extracted with 10% methanol-ethyl acetate. The organic extract is washed with brine, then dried and evaporated. The residue is heated to reflux in n-butanol and pivalic acid for 16 hours. The butanol is evaporated and the residue is pumped dry under vacuum. Fractional crystallization of the residue from 8% methanol-dichloromethane gives 705 mg of 7-chloro-5-(1H-1,2,3-triazol-5-yl)-1,3-dihydro-2H-1,4-benzodiazepin-2-one, mp 246-249 ° C.

Example 3

a) A solution of (2-amino-5-chlorophenyl)-(1H-pyrrol- 2-yl)-methanone (1.5 g) in triflic acid (25 ml) is stirred at 70 ° for 24 hours under argon. After cooling, the solution is poured into 30% NaOH/ice mixture. The mixture is extracted with $CH_2Cl_2$ dried, filtered and evaporated. Chromatography of the residue using a gradient elution system (30% EtOAc/Hex, 40% EtOAc/Hex, 50% EtOAc/Hex) yields 0.69 g of starting material and 0.63 g of (2-amino-5-chlorophenyl)-(1H-pyrrol-3-yl)-methanone, MS Calcd:

220.0400; Found: 220.0403.

b) A solution of 290 mg of the product of a) in 50 ml of $CH_2Cl_2$ and 10 ml of THF containing 600 mg of sodium bicarbonate is stirred with 15 ml of water. To the mixture are added 287 ml of bromoacetylbromide, and stirring is continued. The layers are separated, and the aqueous layer is extracted with $CH_2Cl_2$. The organic extracts are washed with water and brine and then evaporated to dryness. The residue is filtered using 25% ethyl acetate-hexane as eluant to yield 0.45g of 2-bromo-4'-chloro-2'-[(1H-pyrrol-3-yl)carbonyl]acetanilide, mp 141-143°C after recrystallization from $CH_2Cl_2$.

c) To 50 ml of liquid ammonia in a dry-ice bath is added a solution of 0.4 g of the product of b) in 7 ml of THF. The mixture is stirred overnight and the ammonia allowed to evaporate. Solvents are evaporated, and the residue is partitioned between 5 ml of water and 25 ml of ethyl acetate containing 2 ml of methanol. This mixture is stirred. Then, the layers are separated, and the aqueous layer is extracted with the same ethyl acetate-methanol mixture. The organic extract is washed with water and brine, then dried and evaporated. The residue is heated to reflux in n-butanol and pivalic acid. The solvent is evaporated and the residue pumped dry. The residue is dissolved in methanol, treated with charcoal, filtered and concentrated to give 230 mg of 7-chloro-5-(1H-pyrrol-3-yl)-1,3-dihydro-2H-1,4-benzodiazepin-2-one, mp 283-285°C.

Example 4

a) A solution of 11.5 ml of 2.0M ethylmagnesiumbromide in 20 ml of THF is chilled to 0°C and treated with a solution of 1.86 g of 2-methylpyrrole in 7 ml of THF, then stirred at room temperature. The mixture is chilled to 0°C, and a solution of 2.24 g of 2-methyl-6-chloro-4H-3,1-benzoxazin-4-one in 30 ml of THF is added. After warming to room temperature, and stirring, the reaction is quenched by addition of 40 ml of saturated aqueous ammonium chloride. The mixture is partitioned between ether and water, and the aqueous layer is extracted with ether. The ether layer is washed, then dried and evaporated. The residue is combined with a mixture of methanol, 30% sodium hydroxide and water, and heated to reflux for 16 hours. The reaction is cooled in an ice bath, treated with water, and stirred for 0.5 hours. The precipitated solids are washed with water and then dried to give 1.9 g of (2-amino-5-chlorophenyl)-(2-methyl-1H-pyrrol-5-yl)methanone, mp 149-151°C, after recrystallization from ether.

b) To a stirred solution of 0.9 g of the product of a) in 25 ml of $CH_2Cl_2$ containing 1.8 g of sodium bicarbonate is added 0.5 ml of bromacetylbromide. After stirring, water is added, and the mixture is stirred for 0.5 hours. After separation of the layers, the aqueous layer is extracted with $CH_2Cl_2$. The organic layer is washed with water and brine, then dried and evaporated. After filtration, the evaporated residue is crytallized from $CH_2Cl_2$-Hexane to give 1.1 g of 2-bromo-4'-chloro-2'-[2-methyl-1H-pyrrol-5-yl-carbonyl]acetanilide, mp 151-153°C.

c) To 25 ml of liquid ammonia in a dry-ice bath is added a solution of 1.1 g of the compound of b) in 10 ml of $CH_2Cl_2$. The mixture is stirred overnight and the ammonia allowed to evaporate. The residue is partitioned between 30 ml of water and 100 ml of ethyl acetate containing 10 ml of methanol. The mixture is stirred for 1.5 hours. The solid product is collected to give 0.42 g of 2-amino-4'-chloro-2'-[(2-methyl-1H-pyrrol-5-yl)carbonyl]acetanilide. The filtrate is separated, and the aqueous layer is extracted with ethyl acetate. The organic layer is washed with water and brine, then dried and evaporated to give 0.45 g of additional product, mp 200-202°C.

d) A suspension of 0.8 g of the product of c) in 40 ml of n-butanol is heated to reflux for 16 hours. The solvent is evaporated to dryness and the residue is crystallized from dichloromethane to give 0.49 g of 7-chloro-(2-methyl-1H-pyrrol-5-yl)-1,3-dihydro-2H-1,4-benzodiazepin-2-one, m.p. 213-215°C after recrystallization from dichloromethane.

Example 5

a) A solution of 1-(ethoxymethyl)-1H-imidazole (10.0 g) [prepared as in J.A.C.S. 100, 1978, 3918] in THF (150 ml) is cooled to -78°C and treated dropwise with nBuLi (0.0875 mole). The solution is stirred at that temperature for five hours, then treated with a solution of 1,1-(dimethylethyl)-dimethylchlorosilane (13.1 g) in THF (50 ml). After allowing to warm to room temperature, the mixture is quenched with saturated $NH_4Cl$ solution (100 ml), extracted with EtOAc, dried, filtered and evaporated. Flash column chromatography (20% EtOAc/Hexane) yields 6.14 g of 1-(ethoxymethyl)-2-[(1,1-dimethylethyl)dimethylsilyl]-1H-imidazole, MS 239 ($M^+H$).

b) A solution of the product of a) (0.48 g) and THF (5 ml) is cooled to -68°C and then treated with nBuLi (0.86 ml, 2.5M). The solution is maintained at that temperature for 30 minutes, then warmed to -30°C.

After re-cooling to -68°C, the mixture is treated with a solution of 5-chloro-2-nitrobenzaldehyde (0.37 g) in THF (3 ml). The mixture is allowed to warm to room temperature, then quenched with saturated $NH_4Cl$ solution. Extraction with EtOAc is followed by drying, filtration and evaporation. The residue is purified by flash column chromatography (20% EtOAc/Hexane, 40% EtOAc/Hexane) to yield 0.35 g of alpha-(5-chloro-2-nitrophenyl)-(1-[ethoxymethyl-2-(1,1-dimethylethyl)dimethylsilyl]-1H-imidazol-5-yl)methanol, MS calc: 426.1616, Found: 426.1601.

c) A mixture of the product of b) (1.65 g), $MnO_2$ (1.25 g) and $CHCl_3$ (50 ml) is heated to 65°C for 4 hours. After cooling, the reaction is filtered and evaporated. The residue is combined with EtOH and dilute HCl and heated to 65°C for 5 hours. The solution is cooled and neutralized, and the solids are collected to yield 0.90 g of (5-chloro-2-nitrophenyl)-(1H-imidazol-5-yl)-methanone, MS calcd: 251.0098, Found: 251.0135.

d) To a solution of 500 mg of the product of c) in 50 ml of a 1:1:3 mixture of methanol, ethanol, THF are added 100 mg of Raney nickel. The mixture is hydrogenated for 3 hours at atmospheric pressure. The catalyst is removed by filtration and the solvents are evaporated. The residue is purified by flash chromatography using 8.5% $MeOH-CH_2Cl_2$ as eluant. Evaporation gives 198 mg of (2-amino-5-chlorophenyl)(1H-imidazol-5-yl)methanone, MS 221 ($M^+$).

e) To a solution of 154 mg of the product of d) in 10 ml of $CH_2Cl_2$ and 10 ml of THF containing 400 mg of sodium bicarbonate are added 10 ml of water. To the mixture are added 91 ml of bromoacetyl bromide. Then additional 91 ml of bromoacetyl bromide are added and the mixture is stirred for 1 hour. The layers are separated, and the aqueous layer is treated with saturated aqueous sodium bicarbonate and extracted with ethyl acetate. The organic layer is washed with brine, dried and evaporated to give 230 mg of 2-bromo-4'-chloro-2'[(1H-imidazol-5-yl)carbonyl]acetanilide, MS 341 ($M^+$).

f) To 25 ml of liquid ammonia in a dry-ice bath is added a solution of 123 mg of the product of e) in 10 ml of THF. The mixture is stirred overnight and the ammonia allowed to evaporate. The solvents are evaporated, and the residue is partitioned between 10 ml of water and 30 ml of ethyl acetate containing 3 ml of methanol. The aqueous layer is extracted with 10% methanol-ethyl acetate. The organic extracts are washed with brine, dried and evaporated. The residue is heated to reflux in 15 ml of n-butanol containing 10 mg of pivalic acid. The solvent is then evaporated and the residue pumped dry to give 70 mg of 7-chloro-5-(1H-imidazol-5-yl)-1,3-dihydro-2H-1,4-benzodiazepin-2-one, mp 285-290°C, after crystallization from 15% methanol-dichloromethane.

The following galenical compositions containing a compound I or a salt thereof as active ingredients as defined above, can be prepared in a manner known per se:

a) Oral liquid formulation:

| Ingredients | mg/formulation |
|---|---|
| Active ingredient | 20.0 mg |
| Methylparaben | 20.0 mg |
| Sucrose | q.s. |
| Flavoring agent | q.s. |
| Citrate buffer | q.s. |
| Purified water q.s. | 5.0 ml |

b) Tablet formulation:

| Ingredients | mg/tablet |
|---|---|
| Active ingredient | 20 mg |
| Starch | 40 mg |
| Avicel | 80 mg |
| Lactose | 274 mg |
| Magnesium stearate | 2 mg |
| | 416 mg |

c) Soft gelatine capsule formulation:

| Ingredients | mg/capsule |
|---|---|
| Active ingredient | 20 mg |
| Ethoxylated Fatty acids | 500 mg |
| PEG 4000 | 100 mg |
| Vegetable oils q.s. to | 1.0 ml |

## Claims

**1.** Benzodiazepinones of formula

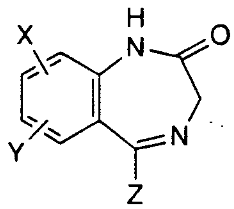

I

wherein X and Y are H, $NO_2$, $NR^1R^2$, halogen, $CF_3$, OH, cyano or lower alkyl or alkoxy;
$R^1$ and $R^2$ are H, lower alkyl, aryl or aralkyl; and
Z is a ring of one of the formulae:

Za 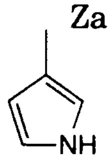 Zb 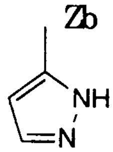 Zc 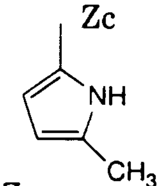 Zd 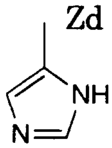

Ze 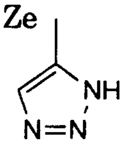 Zf 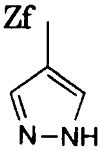 Zg 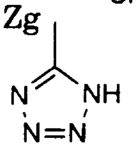

wherein one or more C-atoms can be substituted by H, F, Cl or lower alkyl;
and pharmaceutically acceptable salts, esters and amides thereof.

**2.** The compounds of claim 1, wherein X is H, Y is Cl, and Z is one of the groups $Z^a$ to $Z^e$.

**3.** The compounds of claim 2, wherein Y is Cl in the 7-position, and Z is $Z^a$, $Z^b$ or $Z^e$.

**4.** 7-Chloro-5-(1H-pyrazol-5-yl)-1,3-dihydro-2H-1,4-benzodiazepin-2-one.

**5.** 7-Chloro-5-(1H-1,2,3-triazol-5-yl)-1,3-dihydro-2H-1,4-benzodiazepin-2-one,

**6.** 7-Chloro-5-(1H-pyrrol-3-yl)-1,3-dihydro-2H-1,4-benzodiazepin-2-one.

**7.** 7-Chloro-(2-methyl-1H-pyrrol-5-yl)-1,3-dihydro-2H-1,4-benzodiazepin-2-one.

**8.** 7-Chloro-5-(1H-imidazol-5-yl)-1,3-dihydro-2H-1,4-benzodiazepin-2-one.

**9.** Ketones of formula

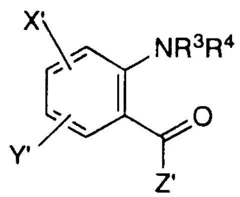

III

wherein X' and Y' are H, $NO_2$, halogen, $CF_3$ or lower alkyl;
Z' is selected from the group consisting of

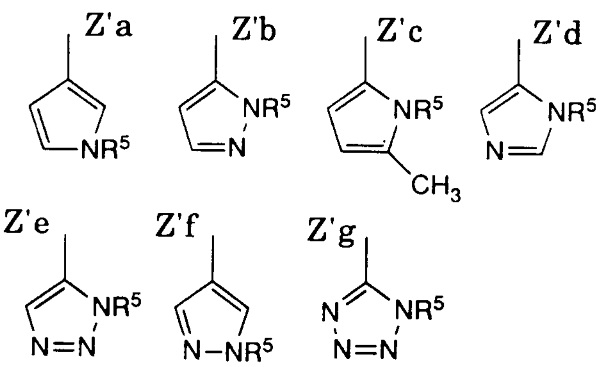

Z'a Z'b Z'c Z'd Z'e Z'f Z'g

where $R^3$, $R^4$ and $R^5$ are a N-protecting group or hydrogen.

**10.** A compound of formula

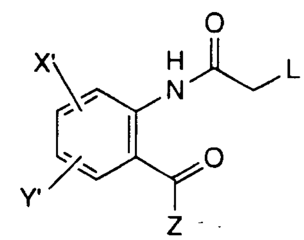

IV'

wherein X' and Y' are as in claim 9, Z is as in claim 1, and L is Br or $NH_2$.

**11.** A compound of formula

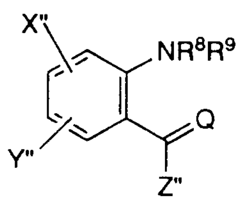

VIII'

wherein X" and Y" are H, $CF_3$, halogen or lower alkyl;
Q is (H, OH) or O,
Z" is as Z' in claim 9 wherein one or more hydrogens on the C-atoms in Z' may be replaced by an unreactive blocking group; and
$R^8$ and $R^9$ are H, O, acyl, trialkylsilyl, alkyldiarylsilyl or t-butoxycarbonyl.

**12.** A compound according to any one of claims 1 to 8 for use as a therapeutically active agent, especially for the treatment or prophylaxis of viral infections, particularly of retroviral infections, such as HIV 1 and/or HIV 2 infections, or for protecting cells against such infections.

**13.** A process for preparing a compound as in claim 1, which comprises cyclizing a compound of formula

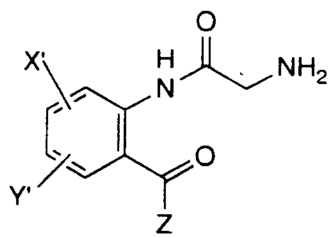

V

wherein X', Y' and Z are as in claim 1 and 9,
by acid-catalysis.

**14.** A medicament, especially for the treatment or prophylaxis of viral infections, particularly of retroviral infections, such as HIV 1 and/or HIV 2 infections, or for protecting cells against such infections, containing as active pharmaceutical ingredient a compound as in any one of claims 1 to 8, and, optionally, a second antiviral agent, especially a reverse transcriptase inhibitor, such as ddC, AZT, a HIV-protease inhibitor, $\alpha$-, $\beta$- and/or $\gamma$-interferon, interleukin-2 and/or GM-CSF.

**15.** The use of a compound as in any one of claims 1 to 8, for the manufacture of a medicament especially for the treatment or prophylaxis of viral infections, particularly of retroviral infections, such as HIV 1 and/or HIV 2 infections, or for protecting cells against such infections.

**Claims for the following Contracting State : ES**

**1.** A process for preparing benzodiazepinones of formula

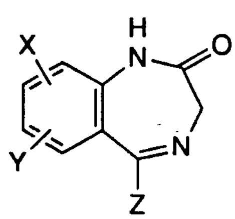

I

wherein X and Y are H, $NO_2$, $NR^1R^2$, halogen, $CF_3$, OH, cyano or lower alkyl or alkoxy;
$R^1$ and $R^2$ are H, lower alkyl, aryl oder aralkyl; and
Z is a ring of one of the formulae:

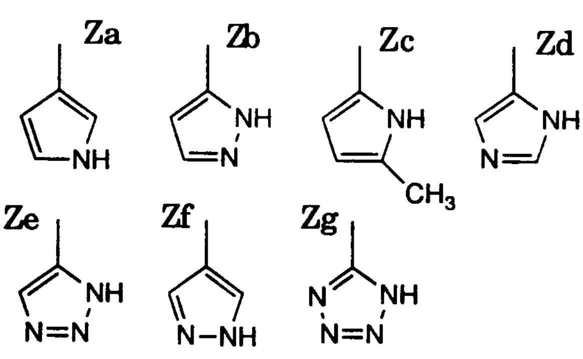

Za Zb Zc Zd

Ze Zf Zg

wherein one or more C-atoms can be substituted by H, F, Cl or lower alkyl;
and pharmaceutically acceptable salts, esters and amides thereof, which process comprises cyclizing a compound of formula

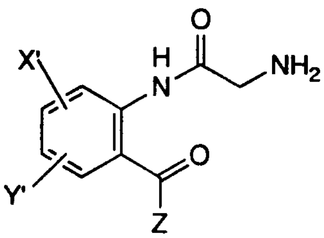

V

wherein X' and Y' are H, $NO_2$, halogen, $CF_3$ or lower alkyl, and Z is as above,
by acid-catalysis.

**2.** The process of claim 1, wherein X is H, Y is Cl, and Z is one of the groups Za to Ze.

**3.** The process of claim 2, wherein Y is Cl in the 7-position, and Z is $Z^a$, $Z^b$ or $Z^e$.

**4.** The process of claim 3, wherein 7-chloro-5-(1H-pyrazol-5-yl)-1,3-dihydro-2H-1,4-benzodiazepin-2-one is prepared.

**5.** The process of claim 3, wherein 7-chloro-5-(1H-1,2,3-triazol-5-yl)-1,3-dihydro-2H-1,4-benzodiazepin-2-one is prepared.

**6.** The process of claim 3, wherein 7-chloro-5-(1H-pyrrol-3-yl)-1,3-dihydro-2H-1,4-benzodiazepin-2-one is prepared.

**7.** The process of claim 3, wherein 7-chloro-(2-methyl-1H-pyrrol-5-yl)-1,3-dihydro-2H-1,4-benzodiazepin-2-one is prepared.

**8.** The process of claim 3, wherein 7-chloro-5-(1H-imidazol-5-yl)-1,3-dihydro-2H-1,4-benzodiazepin-2-one is prepared.

**9.** A process for the preparation of a medicament, especially for the treatment or prophylaxis of viral infections, particularly of retroviral infections, such as HIV 1 and/or HIV 2 infections, or for protecting cells against such infections, which process comprises bringing a benzodiazepinone as in claim 1, into a galenical form.

**10.** The use of a benzodiazepinone as in claim 1, for the manufacture of a medicament especially for the treatment or prophylaxis of viral infections, particularly of retroviral infections, such as HIV 1 and/or HIV 2 infections, or for protecting cells against such infections.

**Claims for the following Contracting State : GR**

**1.** A process for preparing benzodiazepinones of formula

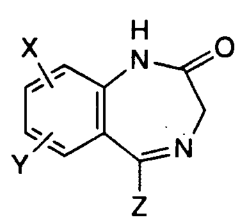

I

wherein X and Y are H, $NO_2$, $NR^1R^2$, halogen, $CF_3$, OH, cyano or lower alkyl or alkoxy;
$R^1$ and $R^2$ are H, lower alkyl, aryl oder aralkyl; and
Z is a ring of one of the formulae:

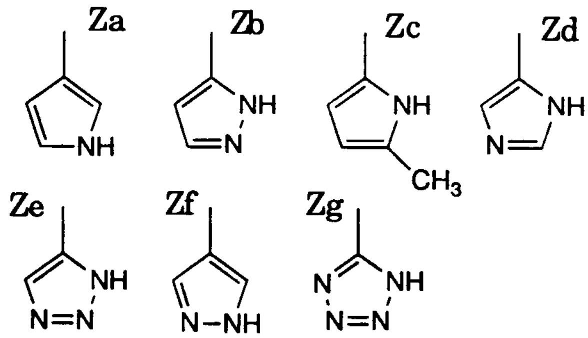

Za Zb Zc Zd Ze Zf Zg

wherein one or more C-atoms can be substituted by H, F, Cl or lower alkyl;
and pharmaceutically acceptable salts, esters and amides thereof, which process comprises cyclizing a compound of formula

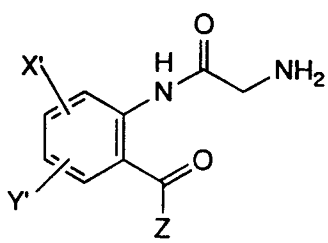

V

wherein X' and Y' are H, $NO_2$, halogen, $CF_3$ or lower alkyl, and Z is as above,
by acid-catalysis.

**2.** The process of claim 1, wherein X is H, Y is Cl, and Z is one of the groups Za to Ze.

**3.** The process of claim 2, wherein Y is Cl in the 7-position, and Z is $Z^a$, $Z^b$ or $Z^e$.

**4.** The process of claim 3, wherein 7-chloro-5-(1H-pyrazol-5-yl)-1,3-dihydro-2H-1,4-benzodiazepin-2-one is prepared.

**5.** The process of claim 3, wherein 7-chloro-5-(1H-1,2,3-triazol-5-yl)-1,3-dihydro-2H-1,4-benzodiazepin-2-one is prepared.

**6.** The process of claim 3, wherein 7-chloro-5-(1H-pyrrol-3-yl)-1,3-dihydro-2H-1,4-benzodiazepin-2-one is prepared.

**7.** The process of claim 3, wherein 7-chloro-(2-methyl-1H-pyrrol-5-yl)-1,3-dihydro-2H-1,4-benzodiazepin-2-one is prepared.

**8.** The process of claim 3, wherein 7-chloro-5-(1H-imidazol-5-yl)-1,3-dihydro-2H-1,4-benzodiazepin-2-one is prepared.

**9.** A process for the preparation of a medicament, especially for the treatment or prophylaxis of viral infections, particularly of retroviral infections, such as HIV 1 and/or HIV 2 infections, or for protecting cells against such infections, which process comprises bringing a benzodiazepinone as in claim 1, into a galenical form.

**10.** The use of a benzodiazepinone as in claim 1, for the manufacture of a medicament especially for the treatment or prophylaxis of viral infections, particularly of retroviral infections, such as HIV 1 and/or HIV 2 infections, or for protecting cells against such infections.

**11.** Ketones of formula

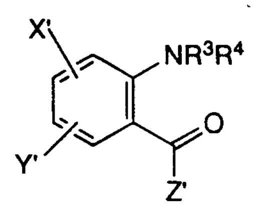

III

wherein X' and Y' are H, $NO_2$, halogen, $CF_3$ or lower alkyl;
Z' is selected from the group consisting of

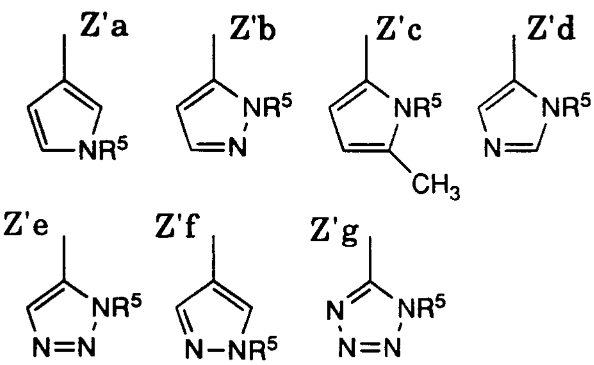

Z'a Z'b Z'c Z'd Z'e Z'f Z'g

where $R^3$, $R^4$ and $R^5$ are a N-protecting group or hydrogen.

**12.** A compound of formula

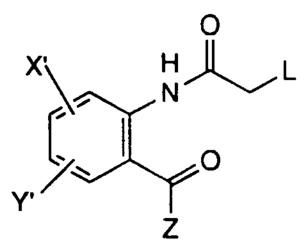

IV'

wherein X' and Y' are as in claim 11,Z is as in claim 1, and L is Br or $NH_2$.

**13.** A compound of formula

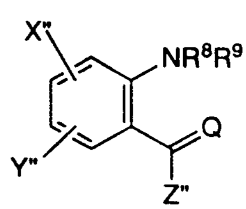

VIII'

wherein X'' and Y'' are H, $CF_3$, halogen or lower alkyl; Q is (H, OH) or O,

Z'' is as Z' in claim 11 wherein one or more hydrogens on the C-atoms in Z' may be replaced by an unreactive blocking group; and

$R^8$ and $R^9$ are H, O, acyl, trialkylsilyl, alkyldiarylsilyl or t-butoxycarbonyl.

European Patent Office

# EUROPEAN SEARCH REPORT

Application Number

EP 91 12 0709
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | EP-A-0 429 868 (F. HOFFMANN-LA ROCHE AG)<br>* claims *<br>--- | 1-3,12, 14 | C07D403/04<br>C07D207/32<br>C07D231/12<br>C07D233/54<br>C07D249/04<br>C07D257/04 |
| X | DE-B-2 017 060 (SUMITOMO CHEMICAL CO., LTD.)<br>* column 5, line 35 - line 39 *<br>* column 12, line 9 - line 10; claim 1 *<br>--- | 1-3, 9-11,13 | |
| D,X | JOURNAL OF HETEROCYCLIC CHEMISTRY<br>vol. 12, February 1975,<br>pages 49 - 57;<br>R. KALISH ET AL.: 'Quinazolines and 1,4-Benzodiazepines ...'<br>* page 49, scheme I *<br>--- | 1-3, 9-11,13 | |
| D,X | US-A-3 405 122 (L. BERGER ET AL.)<br>* column 1, line 41 - column 2, line 51; claims 1,4,6 *<br>--- | 1,2,9 | |
| A | CHEMISTRY AND INDUSTRY<br>18 December 1976,<br>pages 1067 - 1068;<br>M. OGATA ET AL.: 'A convenient synthesis of ...'<br>* page 1067, compound (IV) *<br>--- | 1,2 | TECHNICAL FIELDS SEARCHED (Int. Cl.5 )<br>C07D<br>A61K |
| A | CHEMICAL ABSTRACTS, vol. 113, no. 23, 3 December 1990, Columbus, Ohio, US;<br>abstract no. 211870Y,<br>A.B. TOMCHIN: 'Heterocyclic semicarbazones ...'<br>page 755 ;<br>* compounds VI and VII *<br>& Zh. Org. Khim. 1990, 26(4), 860-73<br>---<br>-/-- | 9-11 | |

The present search report has been drawn up for all claims

| Place of search | Date of completion of the search | Examiner |
|---|---|---|
| BERLIN | 31 MARCH 1992 | CHRISTIAN HASS |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 91 12 0709
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | CHEMICAL ABSTRACTS, vol. 102, no. 15, 15 April 1985, Columbus, Ohio, US; abstract no. 131973S, V.S. VELEZHEVA ET AL.: 'New method for the conversion ...' page 624 ; * compound III * & Khim. Geterotsikl. Soedin. 1984, (12), 1687-8 | 9-11 | |
| A | CHEMICAL ABSTRACTS, vol. 110, no. 23, 5 June 1989, Columbus, Ohio, US; abstract no. 212696W, V.S. VELEZHEVA ET AL.: 'Conversion of ...' page 741 ; * compound II * & Zh. Org. Khim. 1988, 24(7), 1531-40 | 9-11 | |
| A | CHEMICAL ABSTRACTS, vol. 111, no. 17, 23 October 1989, Columbus, Ohio, US; abstract no. 153705U, A.B. TOMCHIN: 'Heterocyclic semicarbazones ...' page 713 ; * compound II * & Zh. Org. Khim. 1988, 24(4), 863-75 | 9-11 | TECHNICAL FIELDS SEARCHED (Int. Cl.5 ) |

-----

The present search report has been drawn up for all claims

| Place of search | Date of completion of the search | Examiner |
|---|---|---|
| BERLIN | 31 MARCH 1992 | CHRISTIAN HASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document